(19) Europäisches Patentamt
European Patent Office
Office européen des brevets

(11) Veröffentlichungsnummer: **0 237 769**
**B1**

(12) **EUROPÄISCHE PATENTSCHRIFT**

(45) Veröffentlichungstag der Patentschrift: **04.04.90**

(21) Anmeldenummer: **87101830.5**

(22) Anmeldetag: **10.02.87**

(51) Int. Cl.$^5$: **C 07 C 49/255,**
C 07 C 43/315, C 07 C 45/71,
C 07 C 41/50, C 25 B 3/02,
C 07 D 249/08

(54) **Benzaldehyd-dialkylacetale.**

(30) Priorität: **20.02.86 DE 3605451**

(43) Veröffentlichungstag der Anmeldung:
**23.09.87 Patentblatt 87/39**

(45) Bekanntmachung des Hinweises auf die Patenterteilung:
**04.04.90 Patentblatt 90/14**

(84) Benannte Vertragsstaaten:
**BE CH DE FR GB IT LI NL**

(56) Entgegenhaltungen:
**EP-A-0 011 712**

(73) Patentinhaber: **BAYER AG**
**D-5090 Leverkusen 1 Bayerwerk (DE)**

(72) Erfinder: **Kaulen, Johannes, Dr.**
**Odenthaler Strasse 10**
**D-5090 Leverkusen 1 (DE)**
Erfinder: **Kranz, Dr. Eckart**
**Am Acker 9**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 237 769 B1

**Beschreibung**

Die Erfindung betrifft neue Benzaldehyd-dialkylacetale, ein Verfahren zu deren Herstellung und deren Verwendung als Zwischenprodukte zur Synthese von Verbindungen mit fungizider Wirksamkeit.

Es ist bereits bekannt geworden, daß man bestimmte in 4-Stellung substituierte Benzaldehyd-dialkylacetale herstellen kann, indem man entsprechende in 4-Stellung substituierte Methyl-benzole elektrochemisch oxidiert (vgl. DE—OS 2 851 732, DE—OS 2 912 058, DE—OS 2 848 397, EP—OS 0 012 240, EP—OS 0 011 712, EP—OS 0 025 883 und US—PS 4 354 904). So sind zum Beispiel auf diese Weise Benzaldehyde-dialkylacetale zugänglich, die in para-Stellung zur Acetalgruppe einen Methoxy-, tert.-Butyloxy-, Phenoxy-, Benzyloxy- oder Allyloxy-Rest enthalten. Eine entsprechende Synthese von Verbindungen mit reaktiven Substituenten am Phenylring is jedoch noch nicht beschrieben worden.

Weiterhin ist bereits bekannt, daß sich fungizid wirksame 1-Hydroxyethyl-triazolyl-Derivate herstellen lassen, indem man substituierte Oxirane mit 1,2,4-Triazol umsetzt (vgl. EP—OS 0 110 048). Die dabei als Ausgangsstoffe benötigten Oxirane sind aber nur durch mehrstufige Synthese zugänglich.

Es wurden nun neue Benzaldehyd-dialkylacetale der Formel

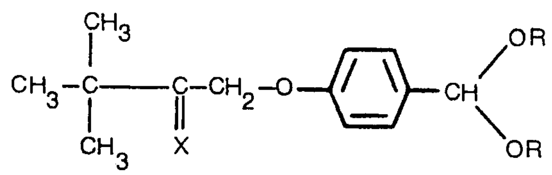

(I)

in welcher
R für Alkyl mit 1 bis 8 Kohlenstoffatomen steht und
X für ein Sauerstoffatom oder eine $CH_2$-Gruppe steht, gefunden.

Weiterhin wurde gefunden, daß man die neuen Benzaldehyd-dialkylacetale der Formel (I) erhält, wenn man substituierte Methylbenzole der Formel

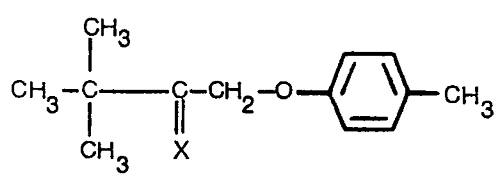

(II)

in welcher
X die oben angegebene Bedeutung hat,
in Gegenwart eines Alkohols der Formel

$$ROH \quad (III)$$

in welcher
R die oben angegebene Bedeutung hat,

und in Gegenwart eines Leitsalzes sowie gegebenenfalls in Gegenwart einer schwer oxidierbaren Aminbase und gegebenenfalls in Gegenwart eines zusätzlichen Verdünnungs-mittels elektrochemisch oxidiert.

Schließlich wurde gefunden, daß sich die neuen Benzaldehyd-dialkylacetale der Formel (I) sehr gut als Zwischen-produkte zur Synthese von fungizid wirksamen 1-Hydroxy-ethyl-triazolyl-Derivaten verwenden lassen.

Es ist als überraschend zu bezeichnen, daß sich die neuen Benzaldehyd-dialkylacetale der Formel (I) nach dem erfindungsgemäßen Verfahren in glatter Reaktion herstellen lassen, denn aufgrund des bekannten Standes der Technik war zu erwarten, daß bei der elektdrochemischen Oxidation von substituierten Methyl-benzolen der Formel (II) auch Nebenreaktionen am Substituenten in para-Stellung zur Methylgruppe eintreten würden. Im übrigen stellen die erfindungsgemäßen Benzäldehyd -dialkylacetale der Formel (I) wertvolle Zwischenprodukte für eine einfach durch-zuführende Synthese von fungizid wirksamen 1-Hydroxyethyl-triazolyl-Derivaten dar.

Die erfindungsgemäßen Benzaldehyd-dialkylacetale sind durch die Formel (I) allegemein definiert. In dieser Formel steht R vorzugsweise für Methyl, Ethyl, n-Propyl, Iso-propyl, n-Butyl, Isobutyl und Octyl. X steht für ein Sauerstoffatom oder eine $CH_2$-Gruppe.

Ganz besonders bevorzugt sind diejenigen Stoffe der Formel (I), in denen R für Methyl oder Ethyl steht und X die oben angegebenen Bedeutungen hat.

Verwendet man tert.-Butyl-(4-methyl-phenoxy)-methyl-keton und Methanol als Ausgangsstoffe,

Kaliumfluorid als Leitsalz und Collidin als schwer oxidierbare Aminbase, so kann der Verlauf des erfindungsgemäßen Verfahrens durch das folgende Formelschema veranschaulicht werden.

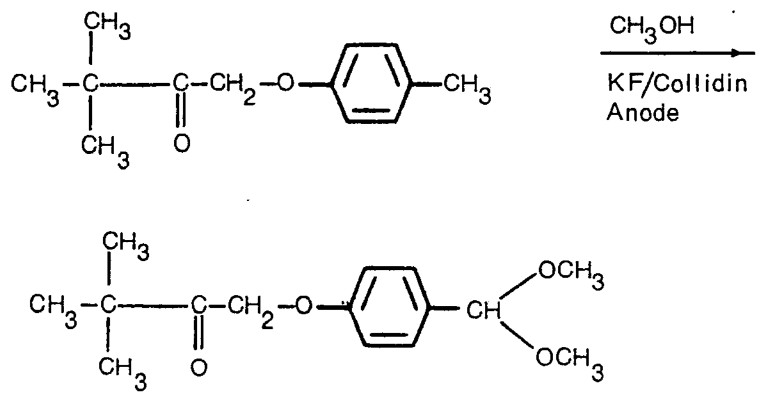


Die bei dem erfindungsgemäßen Verfahren als Ausgangstoffe benötigten substituierten Methylbenzole sind durch die Formel (II) definiert. In dieser Formel steht X für ein Sauerstoffatom oder eine $CH_2$-Gruppe.

Die substituierten Methylbenzole der Formel (II) sind bekannt oder lassen sich nach prinzipiell bekannten Verfahren in einfacher Weise herstellen.

Die bei dem erfindungsgemäßen Verfahren weiterhin als Ausgangsstoffe benötigten Alkohole sind durch die Formel (III) allegmein definiert. In dieser Formel hat R vorzugsweise diejenigen Bedeutungen, die bereits im Zusammenhang mit der Beschreibung der erfindungsgemäßen Stoffe für den Rest R als bevorzugt genannt wurden.

Als Leitsalze können bei der Durchführung des erfindungsgemäßen Verfahrens alle in der Elektrochemie üblichen Leitsalze eingesetzt werden. Vorzugsweise verwendbar sind Leitsalze, die in den zu elektrolysierenden Lösungen löslich und unter den Versuchsbedingungen weitgehend stabil sind. Als Beispiele für derartige Leitsalze seien genannt: Fluoride, wie Kaliumfluorid, ferner Tetrafluoroborate, wie Tetraethylammoniumtetrafluoroborat, außerdem Perchlorate, wie Tetraethylammonium-perchlorat, weiterhin Sulfate, wie Ethyl-tetraethylammonium-sulfat, darüber hinaus Tosylate, wie Tetraethylammonium-tosylat, und auch Alkoholate, wie Natriummethylat.

Als schwer oxidierbare Aminbasen, die dem Elektrolyten bei der Durchführung des erfindungsgemäßen Verfahrens zugesetzt werden können, kommen vorzungsweise Pyridin, Collidin und 2,6-Lutidin in Frage.

Zur Verbesserung der Löslichkeit der substituierten Methylbenzole können bei der Durchführung des erfindungsgemäßen Verfahrens zusätzliche Verdünnungsmittel einegesetzt werden. Vorzugsweise in Betracht kommen dabei Nitrile, wie Acetonitril, ferner Halogenkohlenwasserstoffe, wie Methylenchlorid, außerdem Ether, wie Dimethoxyethan, und weiterhin Ketone, wie Aceton.

Die elektrochemische Oxidation nach dem erfindungsgemäßen Verfahren kann sowohl in einer geteilten als auch in einer ungeteilten Zelle durchgeführt werden. Als Elektrolyt dient dabei eine Lösung aus dem jeweiligen substituierten Methylbenzol, Alkohol und Leitsalz sowie gegebenenfalls schwer oxidierbarer Aminbase und zusätzlichem Verdünnungsmittel.

Die Zusammensetzung des Elektrolyten kann in einem größeren Bereich variiert werden. Im allgemeinen verwendet man Elektrolyten, die zwischen 4 und 40 Gew.-% an substituiertem Methylbenzol der Formel (II), zwischen 50 und 95 Gew.-% an Alkohol der Formel (III), zwischen 0,5 und 10 Gew.-% an Leitsalz, zwischen 0 und 5 Gew.-% an schwer oxidierbarer Aminbase und zwischen 0 und 30 Gew.-% an zusätzlichem Verdünnungsmittel enthalten.

Als Anodenmaterialien können alle unter den Versuchsbedingungen beständigen Elektrodenmaterialien eingesetzt werden. Beispiele für geeignete Anodenmaterialien sind Graphit, graphitgefüllte Kunststoffe, glasartiger Kohlenstoff, Edelmetalle wie Platin und Gold sowie edelmetallbeschichtete Titanelektroden. Als Kathoden werden beispielsweise Graphit-, Eisen-, Stahl-, Blei- oder Edel-metallelektroden eingesetzt.

Die Stromdichten, der Umsatz und die Reaktionstemperaturen können bei der Durchführung des erfindungsgemäßen Verfahrens innerhalb eines größeren Bereiches variiert werden. Im allgemeinen arbeitet man bei Stromdichten zwischen 1 und 20 $A/dm^2$, bei Umsätzen zwischen 2 und 5 F/Mol an substituiertem Methylbenzol der Formel (II) und bei Temperaturen zwischen 0°C und 60°C.

Die Aufarbeitung erfolgt bei dem erfindungsgemäßen Verfahren nach üblichen Methoden. Im allgemeinen geht man so vor, daß man die Elektrolysenausträge destilliert. Überschüssiger Alkohol und gegebenenfalls noch vorhandenes substituiertes Methylbenzol sowie Aminbase und zusätzliche Verdünnungsmittel werden von den gewünschten Endprodukten der Formel (I) durch Destillation abgetrennt und können erneut zur Elektrolyse einegesetzt werden.

Die so erhaltenen erfindungsgemäßen Stoffe der Formel (I) lassen sich, falls erforderlich, durch

fraktionierte Destillation von eventuell noch vorhandenen Verunreinigungen trennen. Das verwendete Leitsalz kann beispielsweise vor der Destillation abfiltriert und zur Elektrolyse zurückgeführt werden.

Wie schon oben erwähnt, eignen sich die erfindungsgemäßen Benzaldehyd-dialkylacetale der Formel (I) als Zwischen-produkte zur Synthese von 1-Hydroxyethyl-triazolyl-Derivaten, welche fungizide Eigenschaften besitzen (vgl. EP—OS 0 110 048).

So lassen sich 1-Hydroxyethyl-triazolyl-Derivate der Formel

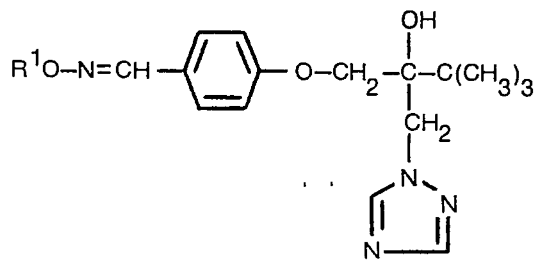

(IV)

in welcher
$R^1$ für Alkyl steht,
herstellen, indem man
a) Bezaldehyd-dialkyl-acetale der Formel

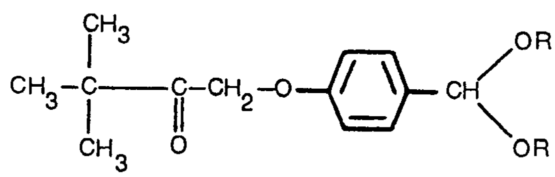

(Ia)

in welcher
R die oben angegebene Bedeutung hat, mit Hydroxylamin-Derivaten der Formel

$$H_2N—OR^1 \quad (V)$$

in welcher
$R^1$ die oben angegebene Bedeutung hat,

oder mit deren Salzen in Gegenwart eines Verdünnungsmittels, wie z.B. Methanol oder Ethanol, sowie gegebenenfalls in Gegenwart eines Säurebindemittels, wie z.B. Natriumacetat, bei Temperaturen zwischen 50 und 100°C umsetzt und die so erhaltenen Verbindungen der Formel

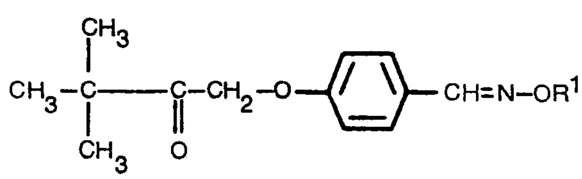

(VI)

in welcher
$R^1$ die oben angegebene Bedeutung hat,
mit Trimethylsulfonium-methylsulfat der Formel

$$(CH_3)_3{}^{\oplus}S \; CH_3SO_4{}^{\ominus} \quad (VII)$$

in Gegenwart eines Verdünnungsmittels, wie z.B. Acetonitril, sowie in Gegenwart einer Base, wie z.B. Natriummethylat, bei Temperaturen zwischen 0°C und 60°C umsetzt und die dabei entstehenden Oxirane der Formel

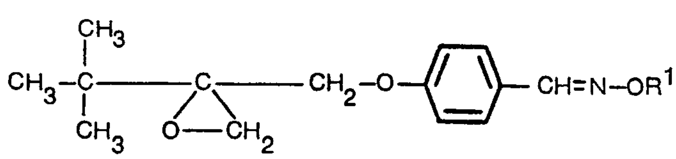

(VIII)

in welcher
$R^1$ die oben angegebene Bedeutung hat,
mit 1,2,4-Triazol der Formel

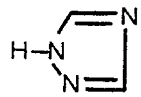

(IX)

oder dessen Alkalimetall-Salzen in Gegenwart eines Verdünnungsmittels, wie z.B. Dimethylformamid, sowie gegebenenfalls in Gegenwart einer Base, wie z.B. Natriumcarbonat, oder Kaliumcarbonat, bei Temperaturen zwischen 60 und 150°C umsetzt,
oder
b) Benzaldehyd-dialkyl-acetale der Formel

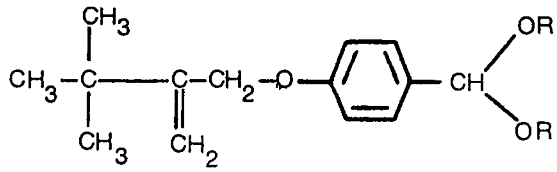

(Ib)

in welcher
R die oben angegebene Bedeutung hat,
mit Hydroxylamin-Derivaten der Formel

$$H_2N—OR^1 \qquad (V)$$

in welcher
$R^1$ die oben angegebene Bedeutung hat,
oder mit deren Salzen in Gegenwart eines Verdünnungsmittels, wie z.B. Methanol oder Ethanol, sowie gegebenenfalls in Gegenwart eines Säurebindemittels, wie z.B. Natriumacetat, bei Temperaturen zwischen 50 und 100°C umsetzt und die so erhaltenen Verbindungen der Formel

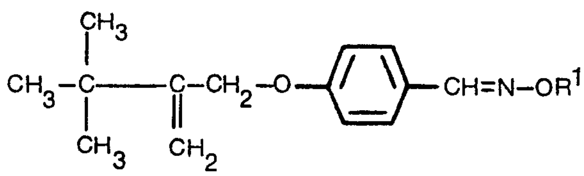

(X)

in welcher
$R^1$ die oben angegebene Bedeutung hat,
mit Persäuren, wie z.B. Peressigsäure, in Gegenwart eines Verdünnungsmittels, wie z.B. Eisessig, bei Temperaturen zwischen 0°C und 50°C epoxidiert und die dabei entstehenden Oxirane der Formel

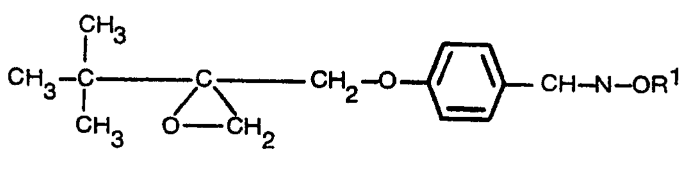

(VIII)

in welcher
$R^1$ die oben angegebene Bedeutung hat,
nach der unter (a) angegebenen Verfahrensweise weiter umsetzt.

Die Herstellung der erfindungsgemäßen Bezaldehyd-dialkyl-acetale wird durch die folgenden Beispiele veranschaulicht.

## Herstellungsbeispiele

### Beispiel 1

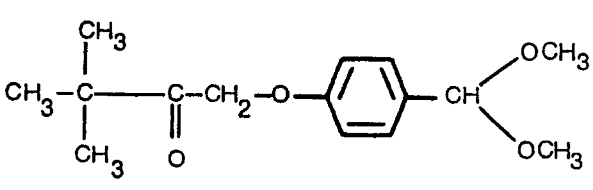

Eine Lösung von 20,6 g (0,1 Mol) tert.-Butyl-(4-methyl-phenoxy)-methyl-keton und 1 g Kaliumfluorid in

60 ml Methanol und 1 ml Collidin wird in einer ungeteilten Zelle an einer Graphit-Anode und einer Edelstahl-Kathode bei einer Stromdichte von 10 $A/dm^2$ und einer Temperatur zwischen 25°C und 35°C bis zum Verbrauch von 0,4 F elektrolysiert. Anschließend engt man den Elektrolyten im Wasserstrahlvakuum ein, saugt das enthaltene Salz ab und destilliert den Rückstand. Man gewinnt dabei in einer Vorfraktion 3,0 g tert.-Butyl-(4-methyl-phenoxy)-methyl-keton vom Siedepunkt 110—120°C/0,1 mbar zurück. Als Hauptfraktion erhält man 15,3 g (57% der Theorie) an tert.-Butyl-[4-(dimethoxy-methyl)-phenoxy]-methyl-keton vom Siedepunkt 125—135°C/0,1 mbar.

Beispiel 2

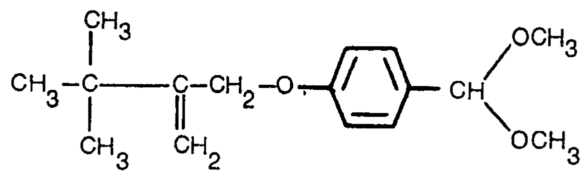

Eine Lösung von 20,6 g (0,1 Mol) 2-tert.-Butyl-3-(4-methyl-phenoxy)-prop-1-en und 1 g Kaliumfluorid in 60 ml Methanol und 1 ml Collidin wird in einer ungeteilten Zelle an einer Graphit-Anode und einer Edelstahl-Kathode bei einer Stromdichte von 10 $A/dm^2$ und einer Temperatur zwischen 25°C und 35°C bis zum Verbrauch von 0,4 F elektrolkysiert. Anschließend engt man den Elektrolyten im Wasserstrahlvakuum ein, saugt das enthaltene Salz ab und destilliert den Rückstand. Man gewinnt dabei in einer Vorfraktion 1,0 g tert.-Butyl-(4-methyl-phenoxy)-prop-1-en vom Siedepunkt 100 bis 110°C/0,1 mbar zurück. Als Hauptfraktion erhält man 18,7 g (71% der Theorie) an 2-tert.-Butyl-3-[4-(dimethoxy-methyl)-phenoxy]-prop-1-en vom Siedepunkt 110 bis 115°C/0,1 mbar.

**Patentansprüche**

1. Benzaldehyd-dialkylacetale der Formel

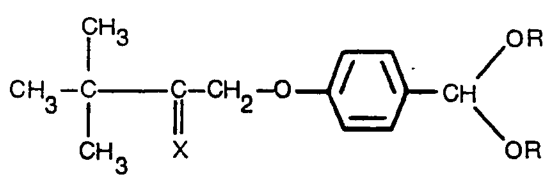

(I)

in welcher
R für Alkyl mit 1 bis 8 Kohlenstoffatomen steht und
X für ein Sauerstoffatom oder eine $CH_2$-Gruppe steht.

2. Verfahren zur Herstellung von Benzaldehyd-dialkyl-acetalen der Formel

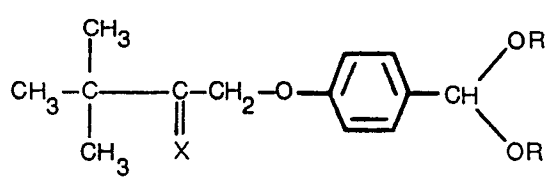

(I)

in welcher
R für Alkyl mit 1 bis 8 Kohlenstoffatomen steht und
X für ein Sauerstoffatom oder eine $CH_2$-Gruppe steht,

dadurch gekennzeichnet, daß man substituierte Methyl-benzole der Formel

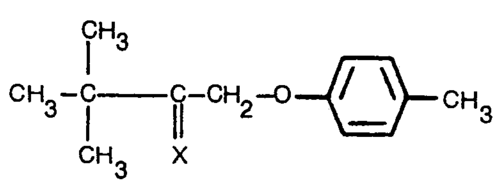

(II)

in welcher

X die oben angegebene Bedeutung hat,
in Gegenwart eines Alkohols der Formel

ROH (III)

in welcher
R die oben angegebene Bedeutung hat,

und in Gegenwart eines Leitsalzes sowie gegebenenfalls in Gegenwart eine schwer oxidierbaren Aminbase und gegebenenfalls in Gegenwart eines zusätzlichen Verdünnungsmittels elektrochemisch oxidiert.

3. Verwendung von Bezaldehyd-dialkylacetalen der Formel (I) als Zwischenprodukte zur Synthese von 1-Hydroxy-ethyl-triazolyl-Derivaten mit fungizider Wirksamkeit.

**Revendications**

1. Dialkylacétals de benzaldéhyde de formule

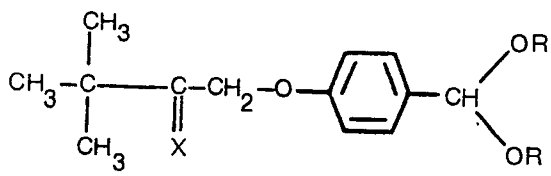

(I)

dans laquelle
R est un groupe alkyle ayant 1 à 8 atomes de carbone et
X est un atome d'oxygène ou un groupe $CH_2$.

2. Procédé de production de dialkylacétals de benzaldéhyde de formule

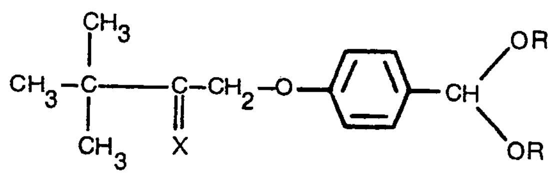

(I)

dans laquelle
R est un groupe alkyle ayant 1 à 8 atomes de carbone, et
X est un atome d'oxygène ou un groupe $CH_2$ caractérisé en ce qu'on oxyde par voie électrochimique des méthylbenzènes substitués de formule

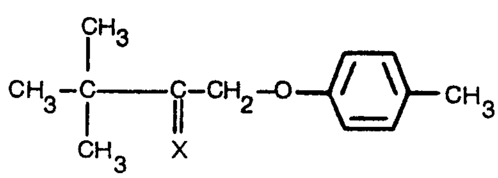

(II)

dans laquelle
x a la définition indiquée ci-dessus, en présence d'un alcool de formule

ROH (III)

dans laquelle
R a las définition indiquée ci-dessus, et en présence d'un sel conducteur ainsi que, le cas échéant, en présence d'une base d'amine difficilement oxydable et en la présence éventuelle d'un diluant supplémentaire.

3. Utilisation de dialkylacétals de benzaldéhyde de formule (I) comme produits intermédiaires pour la synthèse de dérivés 1-hydroxoyéthyl-triazolyliques doués d'activité fongicide.

**Claims**

1. Benzaldehyde dialkyl acetals of the formula

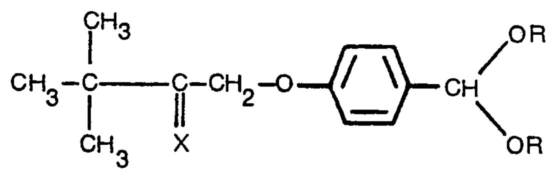

(I)

in which
R represents alkyl with 1 to 8 carbon atoms and
X represents an oxygen atom or a $CH_2$ group.

2. Process for the preparation of benzaldehyde dialkyl acetals of the formula

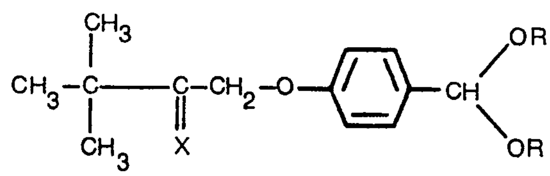

(I)

in which
R represents alkyl with 1 to 8 carbon atoms and
X represents an oxygen atom or a $CH_2$ group,
characterized in that substituted methyl-benzenes of the formula

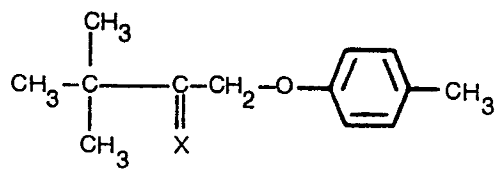

(II)

in which
X has the abovementioned meaning, are subjected to electrochemical oxidation in the presence of an alcohol of the formula

ROH (III)

in which
R has the abovementioned meaning, and in the presence of a conductive salt, and if appropriate in the presence of an amine base which is difficult to oxidize, and if appropriate in the presence of an additional diluent.

3. Use of benzaldehyde dialkyl acetals of the formula (I) as intermediate products for the synthesis of 1-hydroxy-ethyl-triazolyl derivatives with a fungicidal activity.